# EUROPEAN PATENT APPLICATION

(11) **EP 4 083 195 A1**
(43) Date of publication of application: **02.11.2022**
(21) Application number: 20904571.5
(22) Date of filing: 24.12.2020
(51) Int. Cl.: C12N 5/077

(54) **THREE-DIMENSIONAL TISSUE COMPLEX, AND METHOD FOR PRODUCING THREE-DIMENSIONAL TISSUE COMPLEX**

(30) Priority: 26.12.2019 US 201962953887 P
(71) Applicant: The University of Tokyo, Bunkyo-ku, Tokyo 113-8654 (JP)
(72) Inventor: TAKEUCHI, Shoji, Tokyo 113-8654 (JP); MORIMOTO, Yuya, Tokyo 113-8654 (JP); JO, Byeongwook, Tokyo 113-8654 (JP); NIE, Minghao, Tokyo 113-8654 (JP); TAJIMA, Ai, Tokyo 113-8654 (JP)
(74) Representative: Ernest Gutmann - Yves Plasseraud S.A.S.
(86) International application number: PCT/JP2020/048489
(87) International publication number: WO 2021/132478

(57) **Abstract**

A method of producing a 3D tissue composite, comprising: a preparation step in which a multiple number of sheet-shaped first structures containing first cells are prepared, wherein at least one of the multiple number of first structures holds a second structure containing second cells; a stacking step in which the multiple number of first structures are stacked to form a 3D composite; and a culturing step in which the 3D composite is cultured to form a 3D tissue composite containing first tissues formed from the first cells and second tissues formed from the second cells.

## Description

### [Technical Field]

Embodiments of the present invention relates to a 3D (three-dimensional) tissue composite and a method of producing a 3D tissue composite.

Priority is claimed on US Patent Application No. 62/953,887, filed December 26, 2019, the content of which is incorporated herein by reference.

### [Background Art]

In plastic cell culture dishes that are widely used for cell culture, a culture in which cultured cells spread two-dimensionally and proliferate so that they stick to the surface of the dish is obtained. Although such a culture is suitable for the purpose of research such as function elucidation of individual cells and elucidation of proliferation procedures, it is quite different from an environment in which cells form a tissue that is a 3D structure and proliferate and are maintained in a living body, and thus there is a need for a technique that enables cell culture in a state closer to the in-vivo environment.

In addition, in recent years, the development of a method of forming a desired tissue from stem cells such as iPS cells has been promoted, and a culture technique for producing a 3D cell structure has also been useful for constructing a desired 3D tissue using cells differentiated from stem cells. As an example, Japanese Unexamined Patent Application, First Publication No. 2020-141573 discloses a technique for producing 3D tissue structure.

### [Summary of Invention]

### [Problem to be Solved by Invention]

However, in a conventional method, in a case in which a 3D tissue composite composed of two or more types of tissues is produced and co-cultured, it is difficult to accurately position a specific tissue in the 3D composite in order to reproduce the in-vivo environment favorably.

Here, an object of the present invention is to provide a 3D tissue composite having improved handling properties and equivalence to a living body, and a method of producing the 3D tissue composite.

### [Means for Solving Problem]

The present invention includes the following aspects.
[1] A method of producing a 3D tissue composite, comprising: a preparation step in which a multiple number of (i.e. a plurality of) sheet-shaped first structures containing first cells are prepared, wherein at least one of the multiple number of first structures holds a second structure containing second cells; a stacking step in which the multiple number of first structures are stacked to form a 3D composite; and a culturing step in which the 3D composite is cultured to form a 3D tissue composite containing first tissues formed from the first cells and second tissues formed from the second cells.
[2] The method of producing a 3D tissue composite according to [1], wherein the first tissues and the second tissues are co-cultured in the culturing step.
[3] The method of producing a 3D tissue composite according to [1] or [2], wherein the preparation step includes a positioning step in which the second structure is positioned on a substrate, and a gelation step in which a liquid containing the first cells is gelled on the substrate to form the first structure that holds the second structure.
[4] The method of producing a 3D tissue composite according to any one of [1] to [3], wherein the first structures are stacked such that both end portions of the first structures are fixed with anchors in the stacking step.
[5] The method of producing a 3D tissue composite according to any one of [1] to [4], wherein the second structure contains the second tissues formed by culturing the second cells in the preparation step.
[6] The method of producing a 3D tissue composite according to any one of [1] to [5], wherein the second structure has a fiber shape.
[7] The method of producing a 3D tissue composite according to any one of [1] to [6], wherein the second structure has a core part containing the second cells or the second tissues and a shell part that surrounds the core part, and wherein the method further includes a dissolving step in which the shell part is dissolved.
[8] The method of producing a 3D tissue composite according to any one of [1] to [7], wherein the first cells are myoblasts or muscle cells, the first tissues are muscle tissues, the second cells are preadipocytes or adipocytes, and the second tissues are adipose tissues.
[9] A 3D tissue composite, comprising: a sheet-shaped first structure containing first tissues; and a second structure held by the first structure, the second structure containing second tissues, wherein the multiple number of first structures are stacked.
[10] The 3D tissue composite according to [9], wherein first tissues constituting the first structure and second tissues constituting the second structure are in contact with each other.
[11] The 3D tissue composite according to [9] or [10], wherein the second structure has a fiber shape.
[12] The 3D tissue composite according to any one of [9] to [11], wherein the second structure extends in the first structure along a first direction, and wherein the first tissues are muscle tissues containing muscle fibers along the first direction.
[13] The 3D tissue composite according to [12], wherein the first structure has one or more openings extending along the first direction.
[14] The 3D tissue composite according to [13], wherein the opening of one of two adjacent first structures is at a position different from that of the opening of the other of the two adjacent first structures in a second direction orthogonal to the first direction and a stacking direction of the first structure.
[15] The 3D tissue composite according to any one of [9] to [14], wherein the first tissues are muscle tissues, and the second tissues are adipose tissues.

### [Effect of Invention]

According to embodiments of the present invention, it is possible to provide a 3D tissue composite having improved handling properties and equivalence to a living body, and a method of producing the 3D tissue composite.

### [Brief Description of Drawings]

Fig. 1 is a perspective view showing a first production device according to an embodiment.
Fig. 2 is a perspective view showing a first structure having a first shape produced by the first production device according to the embodiment.
Fig. 3 is a perspective view showing a second production device according to an embodiment.
Fig. 4 is a perspective view showing a first structure having a second shape produced by a second production device according to an embodiment.
Fig. 5 is a perspective view showing a third production device according to an embodiment.
Fig. 6 is a flowchart showing a method of producing a 3D tissue composite according to an embodiment.
Fig. 7 is a schematic view illustrating a procedure of producing cell fibers according to an embodiment.
Fig. 8A is a schematic view showing a positioning step in a method of producing a 3D tissue composite according to the embodiment.
Fig. 8B is a schematic view showing a liquid supply step in the method of producing a 3D tissue composite according to the embodiment.
Fig. 8C is a schematic view showing a gelation step in the method of producing a 3D tissue composite according to the embodiment.
Fig. 8D is a perspective view showing a first structure having a first shape that holds a second structure according to an embodiment.
Fig. 8E is a schematic view showing a stacking step in the method of producing a 3D tissue composite according to the embodiment.
Fig. 8F is a schematic view showing a 3D composite according to an embodiment.
Fig. 9 is a schematic view showing the 3D tissue composite according to the embodiment.
Fig. 10 shows images for observing changes over time in fat fibers after arginase was added.
Fig. 11A is an image showing electrical stimulus responsiveness of a muscle-fat composite sheet.
Fig. 11B is a graph showing electrical stimulus responsiveness of a muscle-fat composite sheet.
Fig. 12 shows schematic views showing positions of adipocytes in muscle-fat composite sheets and corresponding bright-field images.
Fig. 13A is an image showing a cut part of a frozen section of a muscle-fat composite sheet.
Fig. 13B is an image of an HE-stained cut surface of a frozen section of a muscle-fat composite sheet.
Fig. 14 is an image showing the results obtained by immunostaining a section of a produced 3D tissue composite.

### [Description of Embodiments]

Hereinafter, a 3D tissue composite and a method of producing a 3D tissue composite according to embodiments will be described with reference to the drawings.

Here, the following embodiment shows one aspect of the present invention, and does not limit the present invention, and can be arbitrarily changed within the technical scope of the present invention. In addition, in the following drawings, in order to allow respective configurations to be easily understood, the sizes and numbers in the structures may be different from those in actual structures.

For illustration, an orthogonal coordinate system containing an x axis, a y axis, and a z axis is defined. The x axis and the y axis are parallel to the horizontal plane, and the z axis is parallel to the vertical direction. Here, the +z direction is defined as upward (that is, the direction opposite to the gravity direction), and the -z direction is defined as downward (that is, the gravity direction). However, the coordinate system is set for convenience of explanation only, and does not limit the present invention.

In this specification, the term "tissue" means a collection of cells in a certain arrangement or morphology. The term "3D structure" means a structure whose structure is not uniform in any of three orthogonal directions (for example, a vertical direction and two horizontal directions orthogonal to each other) in an actual space. The term "3D tissue composite" means an artificially produced 3D structure containing two or more types of tissues.

### [Device for producing 3D tissue composite]

### (First production device for sheet-shaped structure)

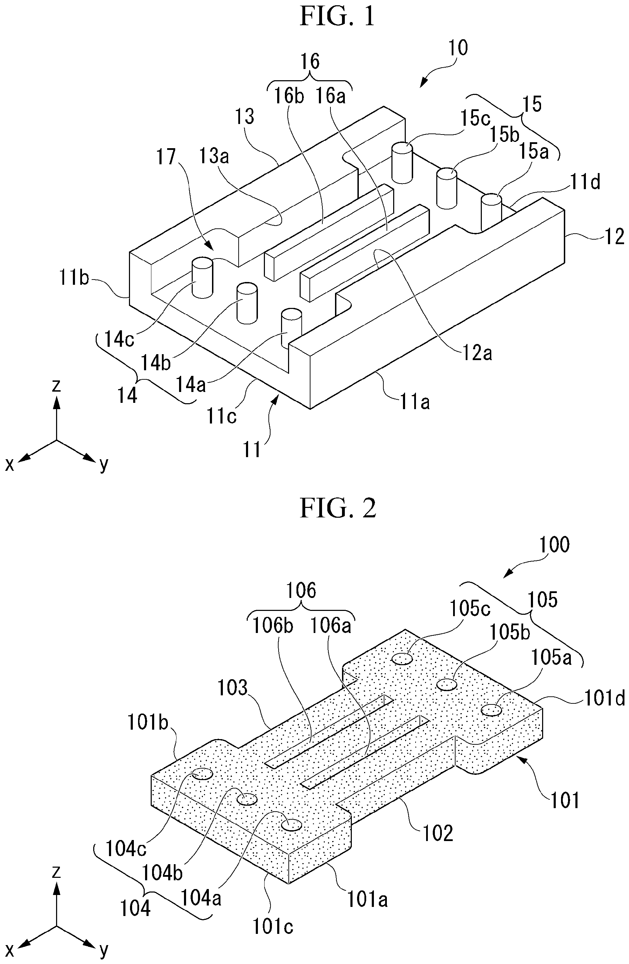

Fig. 1 is a perspective view showing a first production device 10 for producing a sheet-shaped structure.

A first production device 10 includes a base 11, a first wall 12, a second wall 13, a first protrusion 14, a second protrusion 15, and a plate member 16. In addition, the first production device 10 has a concave part 17 defined by the base 11, the first wall 12, and the second wall 13. The first production device 10 is an example of a "substrate."

The base 11 extends along the xy plane, and constitutes the bottom wall of the first production device 10. The base 11 functions as a foundation for producing a first structure. The upper surface of the base 11 functions as a holding surface that holds the first structure.

The first wall 12 and the second wall 13 constitute side walls of the first production device 10 on both sides of the first production device 10 in the y direction. The first wall 12 protrudes upward from the upper surface of the base 11 on the side of a first end 11a of the base 11. The second wall 13 protrudes upward from the upper surface of the base 11 on the side of a second end 11b opposite to the first end 11a of the base 11. The concave part 17 is formed between the first wall 12 and the second wall 13.

The first wall 12 extends along the xz plane and has a first convex part 12a that protrudes in the y direction toward the concave part 17 at a center part in the x direction. Similarly, the second wall 13 extends along the xz plane, and has a second convex part 13a that protrudes in the y direction toward the concave part 17 at a center part in the x direction. Thereby, the concave part 17 has a rectangular shape with recesses on both sides.

The first protrusion 14 and the second protrusion 15 protrude upward from the upper surface of the base 11 on both sides of the first production device 10 in the x direction. The first protrusion 14 protrudes upward from the upper surface of the base 11 on the side of a third end 11c of the base 11. The first protrusion 14 includes three projections 14a, 14b, and 14c arranged in the y direction. The second protrusion 15 protrudes upward from the upper surface of the base 11 on the side of a fourth end 11d opposite to the third end 11c of the base 11. The second protrusion 15 includes three projections 15a, 15b, and 15c arranged in the y direction. Here, the numbers of projections of the first protrusion 14 and the second protrusion 15 are not limited to the above example, and any number of projections may be provided.

The plate member 16 protrudes upward from the upper surface of the base 11 between the first wall 12 and the second wall 13, and between the first protrusion 14 and the second protrusion 15. The plate member 16 extends along the x direction, substantially parallel to the xz plane. The plate member 16 includes two flat plates 16a and 16b. In the present embodiment, in the y direction, the flat plate 16a is disposed between the projection 14a and the projection 14b, and the flat plate 16b is disposed between the projection 14b and the projection 14c. For example, the upper end of the first wall 12, the upper end of the second wall 13, the upper ends of the projections 14a, 14b, 14c, 15a, 15b, and 15c, and the upper ends of the flat plates 16a and 16b are substantially flush with each other (that is, the heights of the upper ends in the z direction are approximately the same). Here, the shape, disposition, and number of plate members 16 are not limited to the above example, and any shape, disposition, and number of plate members may be provided. In addition, a multiple number of plate members along the x direction may be arranged in the x direction, or the plate member may be omitted.

In the present embodiment, the base 11, the first wall 12, the second wall 13, the first protrusion 14, the second protrusion 15, and the plate member 16 constituting the first production device 10 may be integrally formed. However, the configuration of the first production device 10 is not limited to the above example, and some parts of the first production device 10 may be formed as separate components and bonded to each other.

In the present embodiment, the first production device 10 is made of an elastomer, and made of a material, for example, an elastically deformable soft elastic material such as PDMS (polydimethylsiloxane) and silicone rubber, or a hard elastic material such as a resin material and a metal material. That is, the base 11, the first wall 12, the second wall 13, the first protrusion 14, the second protrusion 15, and the plate member 16 which are components of the first production device 10 are made of one or more of the above materials. In the present embodiment, respective components are made of the same material, but one or more components may be made of different materials. However, the material of the first production device 10 is not limited to the above example.

In the present embodiment, at least a part of the surface of the first production device 10 is a cell non-adhesive surface. Preferably, a cell non-adhesive coating is provided on the upper surface of the first production device 10. The cell non-adhesive coating may be, for example, a bovine serum albumin (BSA) coating, and the surface of the first production device 10 may be coated with an MPC (2-methacryloyloxyethyl phosphorylcholine) polymer and then additionally coated with a BSA coating. In addition, any cell non-adhesive material can be used.

In the present embodiment, the first production device 10 is produced by 3D modeling such as optical modeling. However, a method of producing the first production device 10 is not limited to the above example, and any producing method can be used.

In the present embodiment, the first production device 10 is used as a stamp. For example, a liquid or gel-like target material M is placed on a sheet-shaped stamp pad, and the first production device 10 turned upside down (that is, the concave part 17 faces downward) can be stamped from above the target material M. Thereby, the material interposed between the stamp pad and the first production device 10 is molded into a form of the concave part 17. Alternatively, a target material M may be molded by pouring the liquid or gel-like target material M from above the first production device 10 at a normal position (that is, the concave part 17 faces upward as shown in Fig. 1), and placing another flat member thereon to sandwich the target material M. However, a method of using the first production device 10 is not limited to the above example, and the first production device 10 can be used in any other form.

### (First structure having first shape produced by first production device)

Fig. 2 is a perspective view showing a sheet-shaped first structure 100 having a first shape produced by the first production device 10. The first shape is a shape corresponding to the concave part 17 of the first production device 10.

The first structure 100 having a first shape has a main body 101 having a rectangular shape with recesses on both sides, like the concave part 17. That is, the main body 101 having a first shape has a first dent 102 on the side of a first end 101a and a second dent 103 on the side of a second end 101b.

The first structure 100 having a first shape has holes 104a, 104b, and 104c (collectively referred to as first holes 104) corresponding to the projections 14a, 14b, and 14c of the first protrusion 14 on the side of a third end 101c, and holes 105a, 105b, and 105c (collectively referred to as second holes 105) corresponding to the projections 15a, 15b, and 15c of the second protrusion 15 on the side of a fourth end 101d. The first holes 104 and the second holes 105 penetrate the main body 101 in the thickness direction. In addition, the first structure 100 having a first shape has slits 106a and 106b (collectively referred to as slits 106) corresponding to the flat plates 16a and 16b of the plate member 16 between the first dent 102 and the second dent 103 and between the first holes 104 and the second holes 105. The slits 106 penetrate the main body 101 in the thickness direction. The slits 106 are an example of the "opening" of the first structure 100.

In the present embodiment, the first structure 100 contains a matrix component and first cells in the matrix component.

In the present embodiment, the first cells are myoblasts. The myoblasts can be prepared by a known method. For example, the myoblasts may be myoblasts obtained by treating living-body derived muscle tissues with a degrading enzyme, and may be cells induced to differentiate from multipotent stem cells such as ES cells and iPS cells and somatic stem cells. The myoblasts may be cells that are genetically modified or cells that are not genetically modified. Here, the first cells are not limited to the above example, and any one or more cells may be used in place of the myoblasts or in addition to the myoblasts. In addition, muscle cells differentiated from myoblasts may be used. Examples of muscle cells include skeletal muscle cells, smooth muscle cells, and cardiac muscle cells.

The matrix component is not particularly limited, and for example, fibrin, collagen (type I, type II, type III, type V, type XI, etc.), a base membrane component reconstituted from a mouse EHS tumor extract (including type IV collagen, laminin, heparan sulfate proteoglycan and the like) (product name: Matrigel (registered trademark)), gelatin, agar, agarose, glycosaminoglycan, hyaluronic acid, proteoglycan and the like may be exemplified.

The first structure 100 may contain a component other than the first cells and the matrix component. For example, the first structure 100 may contain a medium component (for example, amino acid, vitamins, inorganic salts, glucose, etc.), a coagulant (thrombin, etc.), a serum component, an antibiotic, and any other additive. In particular, in a case in which a tissue composite for artificial meat is produced, the first structure 100 may contain, for example, various amino acids, vitamins, inorganic salts and the like.

The cell density of the first cells in the first structure 100 is, for example, 1.0×10⁶ cells/mL or more, preferably 1.0×10⁷ cells/mL or more and 1.0×10⁹ cells/mL or less, more preferably 2.0×10⁷ cells/mL or more and 5.0×10⁸ cells/mL or less, and still more preferably 5.0×10⁷ cells/mL or more and 1.0×10⁸ cells/mL or less.

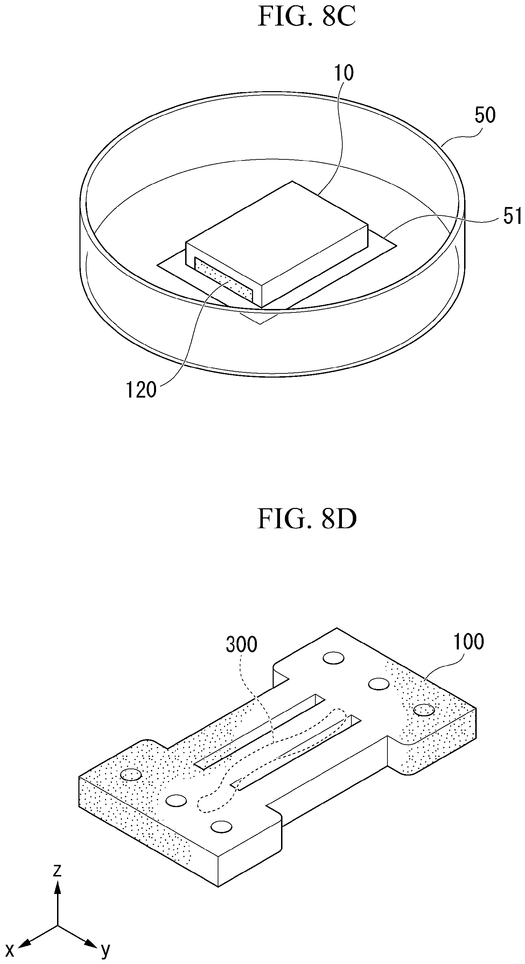

The sheet-shaped first structure 100 can hold a second structure 300 containing second cells or second tissues (refer to Fig. 8D). In the present embodiment, after the second structure 300 is placed on the upper surface of the base 11 in the concave part 17, the concave part 17 is filled with a raw material liquid of the first structure 100 so that the second structure 300 is surrounded by the first structure 100, the matrix component is gelled, and thus the first structure 100 holding the second structure 300 can be obtained (details will be described below).

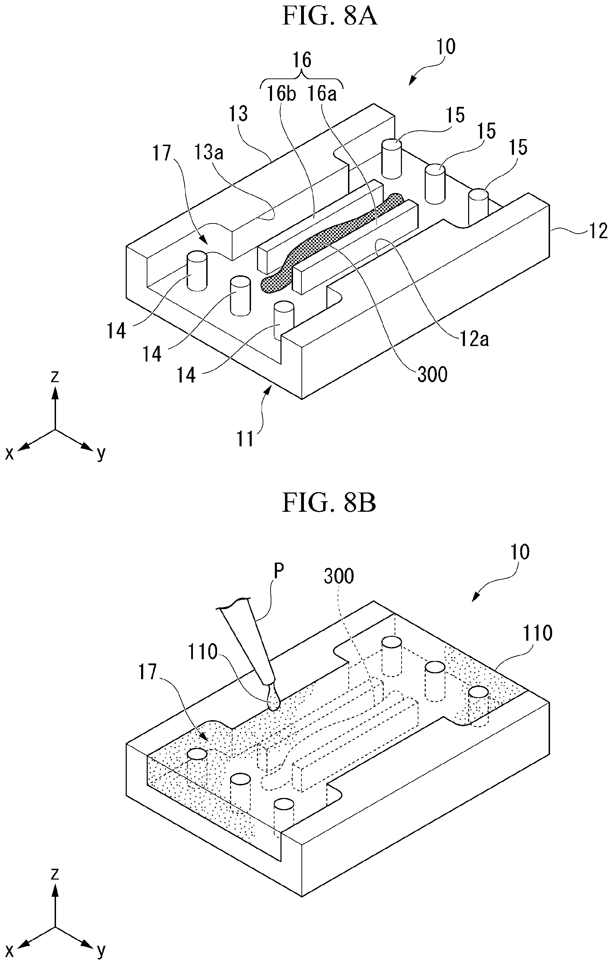

In the present embodiment, the second cells are preadipocytes or adipocytes, and form a fibrous cell mass (hereinafter the fibrous cell mass is also referred to as a "cell fiber") (refer to Fig. 8A). The second tissue is an adipose tissue and forms a fibrous tissue or tissue mass (hereinafter the fibrous tissue or the tissue mass is also referred to as a "tissue fiber"). The second structure is a structure in which the above cell fiber or tissue fiber is wrapped in a gel-like shell part as a core part. For example, the second structure contains an adipose tissue (second tissue) and also contains adipocytes (second cells) constituting the adipose tissue.

The cell density of the second cells in the core part of the second structure is, for example, 1.0×10⁶ cells/mL or more, preferably 1.0×10⁷ cells/mL or more and 1.0×10⁹ cells/mL or less, and more preferably 5.0×10⁷ cells/mL or more and 5.0×10⁸ cells/mL or less.

The preadipocytes or adipocytes constituting the cell mass can be prepared by a known method. For example, the preadipocytes or adipocytes may be adipocytes obtained by treating a living-body derived adipose tissue with a degrading enzyme, and may be cells induced to differentiate from multipotent stem cells such as ES cells and iPS cells and somatic stem cells. The adipocytes may be cells that are genetically modified or cells that are not genetically modified. Here, the second cells are not limited to the above example, and any one or more cells may be used in place of the adipocytes or in addition to the adipocytes.

The shell part of the second structure 300 may contain a dissociative hydrogel. Examples of dissociative hydrogels include hydrogels that gel in the presence of metal ions, enzyme-soluble hydrogels, temperature-responsive hydrogels, pH-responsive hydrogels, photoresponsive hydrogels, and magnetic field-responsive hydrogels. Hydrogels that gel in the presence of metal ions can be dissociated by removing the metal ions. Examples of metal ions include calcium ions, magnesium ions, strontium ions, barium ions, sodium ions, and potassium ions. The enzyme-soluble hydrogel can decompose and dissolve molecules constituting the gel according to the action of an enzyme. The temperature-responsive hydrogel can change the solation and gelation state by changing the temperature beyond the phase transition temperature. The pH-responsive hydrogel can be dissociated by changing the pH. The photoresponsive hydrogel can be dissociated, for example, by emitting light having a certain wavelength. The magnetic field-responsive hydrogel can be dissociated, for example, by changing the magnetic field.

Examples of hydrogels that gel in the presence of metal ions include arginate gels that gel in the presence of divalent or trivalent metal ions, carrageenan gels that gel in the presence of calcium ions or potassium ions, and acrylic acid-based synthetic gels that gel in the presence of sodium ions. Examples of enzyme-soluble hydrogels include arginate gels, chitosan gels, cellulose gels, collagen gels, and fibrin gels. Examples of temperature-responsive hydrogels include a temperature-responsive hydrogel (trade name: Mebiol gel) obtained by cross linking poly(N-isopropylacrylamide) with polyethylene glycol, methyl cellulose, hydroxypropyl cellulose, a copolymer of polylactic acid and polyethylene glycol, and a triblock copolymer of polyethylene glycol and polypropylene oxide (trade name: Pluronic (registered trademark), Poloxamer). Examples of pH-responsive hydrogels include arginate gels, chitosan gels, carboxymethyl cellulose gels, and acrylic acid-based synthetic gels. Examples of photoresponsive hydrogels include synthetic gels in which azobenzene and cyclodextrin are combined in the framework, gels composed of super molecules with fumaric acid amides as a spacer, and gels that are cross-linked or bonded via a nitrobenzyl group. Examples of magnetic field-responsive hydrogels include gels composed of crosslinked poly(N-isopropylacrylamide) containing magnetic particles.

A method of forming the second structure 300 and a method of forming a composite containing a first structure and a second structure will be described below in detail.

### (Second production device for sheet-shaped structure)

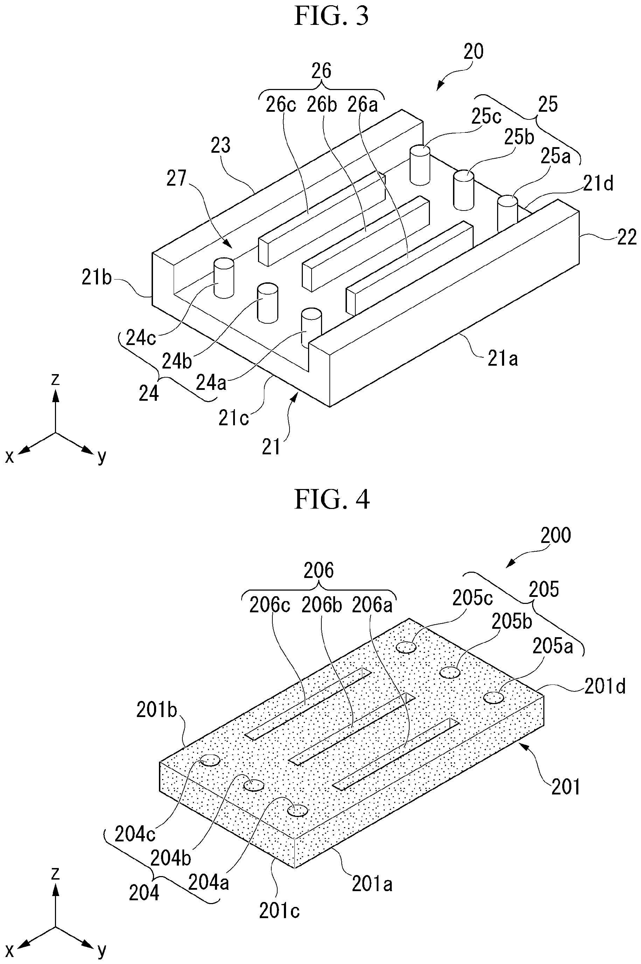

Fig. 3 is a perspective view showing a second production device 20 for producing a sheet-shaped structure.

The second production device 20 includes a base 21, a first wall 22, a second wall 23, a first protrusion 24, a second protrusion 25, and a plate member 26. In addition, the second production device 20 has a concave part 27 defined by the base 21, the first wall 22, and the second wall 23. The second production device 20 is an example of the "substrate." The second production device 20 has basically the same configuration as the first production device 10 except for points described below.

The first wall 22 and the second wall 23 of the second production device 20 are disposed on the side of a first end 21a and on the side of a second end 21b of the base 21, respectively, and have a substantially uniform thickness in the y direction. That is, unlike the first wall 12 and the second wall 13 of the first production device 10, in the first wall 22 and the second wall 23, the first convex part 12a and the second convex part 13a are not provided. Thereby, the concave part 27 has a simple rectangular shape having no dent. However, the shapes of the first wall 22 and the second wall 23 of the second production device 20 are not limited to the above example, and non-flat parts such as a convex part and concave part having shapes different from those of the first convex part 12a and the second convex part 13a of the first production device 10 may be formed on the first wall 22 and the second wall 23.

Like the first protrusion 14 of the first production device 10, the first protrusion 24 of the second production device 20 is disposed between the first wall 22 and the second wall 23 on the side of a third end 21c of the base 21 and includes three projections 24a, 24b, and 24c arranged in the y direction. Like the second protrusion 15 of the first production device 10, the second protrusion 25 is disposed between the first wall 22 and the second wall 23 on the side of a fourth end 21d of the base 21 and includes three projections 25a, 25b, and 25c arranged in the y direction.

The plate member 26 of the second production device 20 includes three flat plates 26a, 26b, and 26c extending along the x direction, substantially parallel to the xz plane. In a case in which the first production device 10 and the second production device 20 overlap in the z direction, the position of the plate member 26 of the second production device 20 is at least partially displaced in the y direction from the position of the plate member 16 of the first production device 10. Preferably, the positional relationship between the first protrusion 24, the second protrusion 25 and the plate member 26 of the second production device 20 in the y direction is different from the positional relationship between the first protrusion 14, the second protrusion 15 and the plate member 16 of the first production device 10 in the y direction. Thereby, it is possible to produce two types of sheet-shaped structures having different slit positions in the y direction. In the present embodiment, the positions of the projection 24a, the flat plate 26a, and the projection 25a in the y direction are substantially the same, the positions of the projection 24b, the flat plate 26b, and the projection 25b in the y direction are substantially the same, and the positions of the projection 24c, the flat plate 26c, and the projection 25c in the y direction are substantially the same. However, the disposition of the plate member 26 is not limited to the above example, and the positions of the projection and the flat plate in the y direction may be displaced.

Like the first production device 10, for example, the upper end of the first wall 22, the upper end of the second wall 23, the upper ends of the projections 24a, 24b, 24c, 25a, 25b, and 25c, and the upper ends of the flat plates 26a, 26b, and 26c are substantially flush with each other (that is, the heights of the upper ends in the z direction are approximately the same).

In the present embodiment, the second production device 20 has substantially the same size as the first production device 10. However, the size of the second production device 20 is not limited to the above example, and may be larger than the first production device 10 or smaller than the first production device 10 as necessary.

### (First structure having second shape produced by second production device)

Fig. 4 is a perspective view showing a sheet-shaped first structure 200 having a second shape produced by the second production device 20. The second shape is a shape corresponding to the concave part 27 of the second production device 20. The first structure 200 having a second shape has basically the same configuration as the first structure 100 having a first shape except for points described below.

Like the concave part 27, the first structure 200 having a second shape has a main body 201 having a simple rectangular shape having no dent.

The first structure 200 having a second shape has holes 204a, 204b, and 204c (collectively referred to as first holes 204) corresponding to the projections 24a, 24b, and 24c of the first protrusion 24 on the side of a third end 201c, and has holes 205a, 205b, and 205c (collectively referred to as second holes 205) corresponding to the projections 25a, 25b, and 25c of the second protrusion 25 on the side of a fourth end 201d. The first holes 204 and the second holes 205 penetrate the main body 201 in the thickness direction. In addition, the first structure 200 having a second shape has slits 206a, 206b, and 206c (collectively referred to as slits 206) corresponding to the flat plates 26a, 26b, and 26c of the plate member 26 between the first holes 204 and the second holes 205. The slits 206 penetrate the main body 201 in the thickness direction.

In the present embodiment, the first structure 200 having a second shape contains the same matrix component as that of the first structure 100 having a first shape and first cells in the matrix component. In addition, like the first structure 100, the sheet-shaped first structure 200 can hold the second structure 300 containing second cells or second tissues.

### (Third production device for 3D structure)

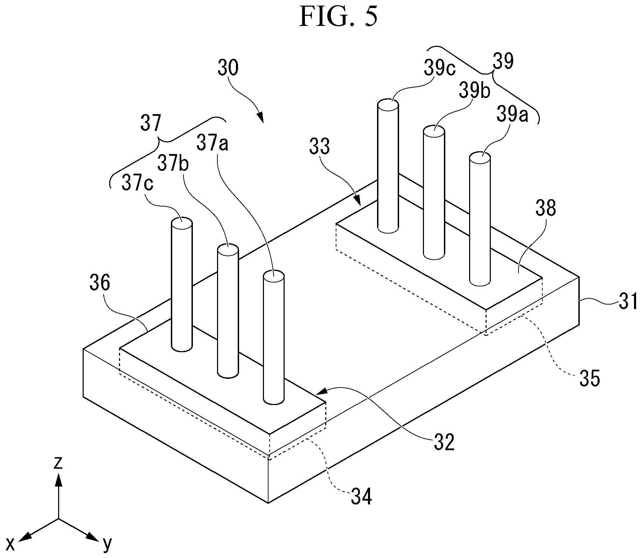

Fig. 5 is a perspective view showing a third production device 30 for producing a 3D structure.

The third production device 30 includes a base 31, a first anchor member 32, and a second anchor member 33. The first anchor member 32 and the second anchor member 33 can fix one or more first structures 100 and 200.

The base 31 is a rectangular member extending along the xy plane. On the upper surface of the base 31, a first anchor accommodation part 34 and a second anchor accommodation part 35 formed as a rectangular recessed part are provided.

The first anchor member 32 includes a first anchor base 36 and first anchors 37. The first anchor base 36 has a rectangular shape having a size corresponding to the first anchor accommodation part 34 so as to be accommodated in the first anchor accommodation part 34, and functions as a foundation of the first anchors 37. The first anchors 37 include three rod-shaped anchors 37a, 37b, and 37c protruding upward from the first anchor base 36. The anchors 37a, 37b, and 37c have columnar shapes having a circular cross-sectional shape, and are formed to have, for example, a diameter of about 100 µm to 1 mm and a length of about 1 mm to 10 mm. In the present embodiment, the anchors 37a, 37b, and 37c are provided at positions corresponding to the first protrusion 14 of the first production device 10 and the first protrusion 24 of the second production device 20. That is, the positions of the anchors 37a, 37b, and 37c correspond to the positions of the first holes 104 of the first structure 100 having a first shape and the first holes 204 of the first structure 200 having a second shape. Here, the number, disposition, shape, size and the like of anchors are not limited to the above examples, and the anchors may be arranged, for example, in a grid form or zigzag form. In addition, the anchors 37a, 37b, and 37c may be formed such that they can puncture a part in which the holes of the first structures 100 and 200 are not formed.

The second anchor member 33 includes a second anchor base 38 and second anchors 39. The second anchor base 38 has a rectangular shape having a size corresponding to the second anchor accommodation part 35 so as to be accommodated in the second anchor accommodation part 35, and functions as a foundation of the second anchors 39. The second anchors 39 include three rod-shaped anchors 39a, 39b, and 39c protruding upward from the second anchor base 38. The anchors 39a, 39b, and 39c may have the same shape and size as the anchors 37a, 37b, and 37c. In the present embodiment, the anchors 39a, 39b, and 39c are provided at positions corresponding to the second protrusion 15 of the first production device 10 and the second protrusion 25 of the second production device 20. That is, the positions of the anchors 39a, 39b, and 39c correspond to the positions of the second holes 105 of the first structure 100 having a first shape and the second holes 205 of the first structure 200 having a second shape. Here, the number, disposition, shape, size, and the like of anchors are not limited to the above examples. In addition, the anchors 39a, 39b, and 39c may be formed such that they can puncture a part in which the holes of the first structures 100 and 200 are not formed.

Since the first anchors 37 and the second anchors 39 are disposed as described above, the first anchors 37 pass through the first holes 104 of the first structure 100 having a first shape and the second anchors 39 pass through the second holes 105, and thereby the first structure 100 having a first shape can be fixed with the first anchor member 32 and the second anchor member 33. Similarly, the first anchors 37 pass through the first holes 204 of the first structure 200 having a second shape and the second anchors 39 pass through the second holes 205, and thereby the first structure 200 having a second shape can be fixed with the first anchor member 32 and the second anchor member 33. In addition, by disposing a multiple number of first structures 100 and 200 on the third production device 30, the multiple number of first structures 100 and 200 can be stacked.

In the first anchor member 32 and the second anchor member 33, at least the surfaces of the first anchor base 36 and the second anchor base 38 and the surfaces of the first anchors 37 and the second anchors 39 are coated with a material that does not adversely affect cells, for example, polyparaxylylene (so-called parylene).

### [Method of producing 3D tissue composite]

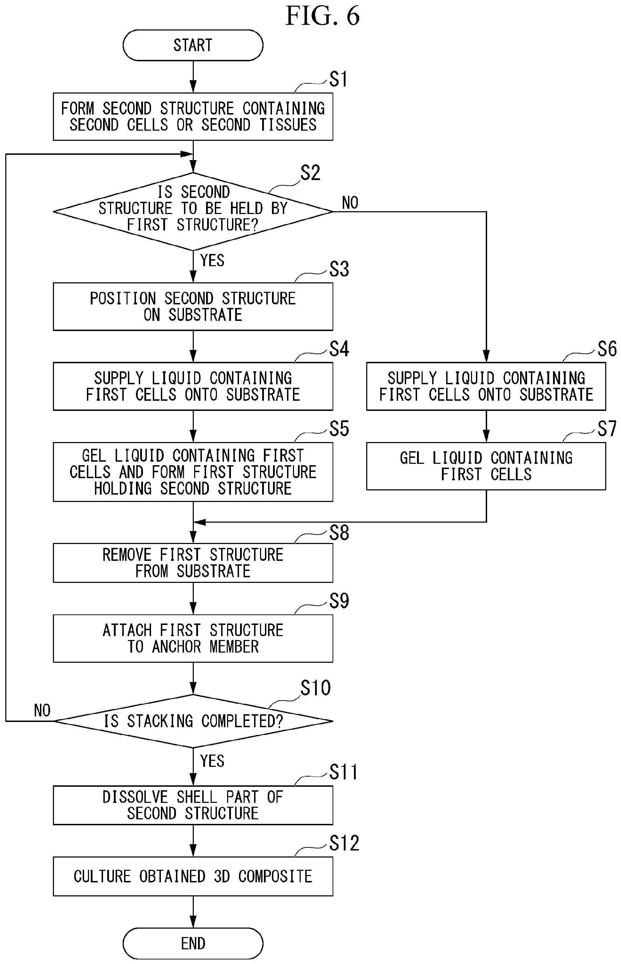

Next, a method of producing a 3D tissue composite according to the present embodiment will be described with reference to Fig. 6 to 9.

A method of producing a 3D tissue composite according to the present embodiment includes a preparation step in which a multiple number of sheet-shaped first structures containing first cells are prepared, which is a preparation step in which at least one of the multiple number of first structures holds a second structure containing second cells, a stacking step in which the multiple number of first structures are stacked to form a 3D composite, and a culturing step in which the 3D composite is cultured to form a 3D tissue composite containing first tissues formed from the first cells and second tissues formed from the second cells. Preferably, the preparation step includes a positioning step in which the second structure is positioned on a substrate and a gelation step in which a liquid containing the first cells is gelled on the substrate to form the first structure that holds the second structure.

Fig. 6 is a flowchart showing a method of producing a 3D tissue composite according to the present embodiment. Hereinafter, details will be described with reference to the flowchart. Hereinafter, a case in which the first structure 100 having a first shape is produced mainly using the first production device 10 will be described, but the same applies to a case in which the first structure 200 having a second shape is produced using the second production device 20.

First, the second structure 300 containing second cells or second tissues is formed (Step S1). In the present embodiment, the second cells are preadipocytes or adipocytes forming a cell fiber, the second tissue is an adipose tissue forming a tissue fiber, and the second structure 300 is a core-shell structure having a core part 301 containing a cell fiber of second cells or a tissue fiber of second tissues, and a shell part 302 that surrounds the core part 301.

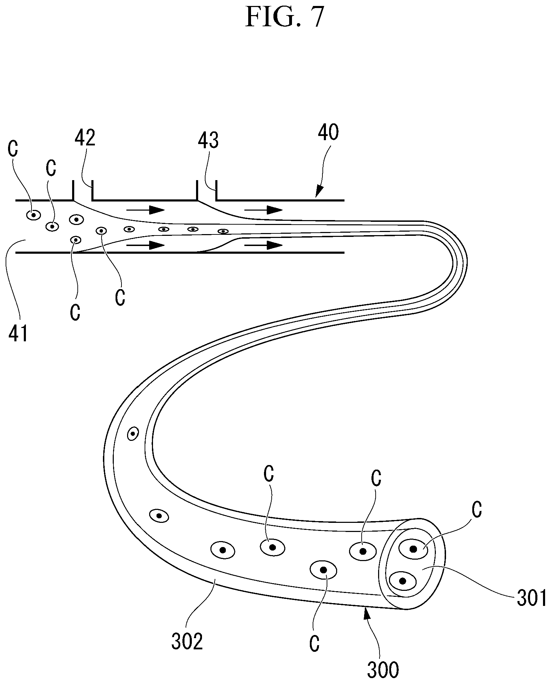

A method of producing the second structure 300 is not particularly limited, but it can be easily produced using, for example, a double coaxial microfluidic device 40 as shown in Fig. 7. Fig. 7 is a schematic view illustrating a procedure of producing the second structure 300 according to the present embodiment. As an example, a case in which a collagen solution is used as a material of the core part 301, and a sodium alginate solution before cross linking is used as a material of the shell part 302 will be described.

First, a collagen solution containing cells C is introduced and injected from an inlet 41 of the microfluidic device 40. In addition, a sodium alginate solution before cross linking is introduced and injected from an inlet 42 of the microfluidic device 40. In addition, a calcium chloride solution is introduced and injected from an inlet 43 of the microfluidic device 40. Thus, the sodium alginate solution of the shell part 302 gels, and the second structure 300 in which the shell part 302 is an arginate gel can be produced. In addition, by heating the second structure 300 at about 37°C for about several minutes to 1 hour, collagen solution containing cells C of the core part 301 can be gelled. In this manner, the second structure 300 including the cell fiber in the core part 301 can be produced.

The injection speed of the solution at the inlets 41 and 42 is not particularly limited, but in a case in which the diameter of the microfluidic device 40 is about 50 µm to 2 mm, the injection speed may be about 10 to 500 µL/min. By adjusting the injection speed of the solution at the inlets 41 and 42, the diameter of the core part 301 and the coating thickness of the shell part 302 can be appropriately adjusted. The injection speed of the solution at the inlet 43 is not particularly limited, but may be, for example, about 1 to 10 mL/min.

The outer diameter of the second structure 300 is not particularly limited, and may be, for example, 10 µm to 2 mm, for example, 20 µm to 2 mm, and for example, about 50 µm to 1 mm. The thickness of the shell part 302 is not particularly limited, and may be, for example, 5 µm to 1 mm, for example, 10 µm to 1 mm, and for example, about 50 µm to 500 µm. The length of the second structure 300 is not particularly limited, and may be about several mm to several m. Examples of cross-sectional shapes of the second structure 300 include a circular shape, an elliptical shape, and a polygonal shape such as a quadrangular shape and a pentagonal shape.

By culturing the cell fiber of the second structure 300 in a culture solution, cells can proliferate. The cell fiber can be cultured for several months by appropriately replacing the culture solution. In a case in which second cells constituting the cell fiber of the core part 301 proliferate to form second tissues by culturing, the second structure 300 contains the tissue fiber in the core part 301.

The core part 301 may contain various growth factors suitable for maintaining, proliferating, or function expressing cells C, for example, an epidermal growth factor (EGF), a platelet derived growth factor (PDGF), a transforming growth factor (TGF), an insulin-like growth factor (IGF), a fibroblast growth factor (FGF), and a nerve growth factor (NGF). In a case in which a growth factor is contained, an appropriate concentration can be selected according to the type of the growth factor.

Next, it is determined whether the first structure 100 to be produced holds the second structure 300 (Step S2). For example, in a case in which both the first structure 100 holding the second structure 300 and the first structure 100 not holding the second structure 300 are used to form a final 3D tissue composite, determination in Step S2 is performed according to whether the first structure 100 to be stacked next holds the second structure 300. The determination may be performed by a determination subject such as a user each time, or may be automatically performed according to a preset flow or the like.

In a case in which it is determined that the first structure 100 holds the second structure 300 (Yes in Step S2), the second structure 300 produced in Step S1 is positioned on the holding surface of the first production device 10 (Step S3). As shown in Fig. 8A, the second structure 300 may be disposed on the upper surface of the base 11 (holding surface) along a groove formed by being partitioned by the plate member 16 in the x direction. That is, the second structure 300 may be positioned between the first convex part 12a and the flat plate 16a, between the flat plate 16a and the flat plate 16b, or between the flat plate 16b and the second convex part 13a. In this manner, the plate member 16 can function as a positioning member for positioning the second structure 300. Here, a method of positioning the second structure 300 is not limited to the above example, and the second structure 300 may be disposed at any position of the concave part 17. In addition, two or more second structures 300 may be disposed.

Next, a liquid 110 containing first cells is supplied onto the holding surface of the first production device 10 (Step S4). As shown in Fig. 8B, for example, the liquid 110 is supplied to the concave part 17 using a pipette P or the like. The liquid 110 is a raw material of the first structure 100, and contains a liquid before gelation of the first cells and the matrix component. The matrix component is as described above for the first structure 100.

In the present embodiment, as the liquid 110 before gelation of the matrix component, a mixed liquid A containing thrombin and a growth medium and a mixed liquid B containing fibrinogen, Matrigel (registered trademark) and a growth medium are used. That is, by mixing the mixed liquid A and the mixed liquid B, the matrix component is gelled. In the present embodiment, the mixed liquid A and the mixed liquid B are mixed before they are supplied to the concave part 17, but the mixed liquid A and the mixed liquid B may be separately supplied to the concave part 17 and mixed in the concave part 17.

Next, by gelling the liquid 110, the first structure 100 holding the second structure 300 is formed (Step S5). Gelation may be performed by any known method. In the present embodiment, since gelation starts when the mixed liquid A and the mixed liquid B are mixed, the liquid 110 is supplied to the concave part 17 while gelation of the liquid 110 proceeds (Fig. 8B). Since the liquid 110 fills the concave part 17 in which the second structure 300 is disposed, the second structure 300 is wrapped in the liquid 110. Then, the first production device 10 in a state in which the concave part 17 is filled with the liquid 110 is placed on a sheet-shaped stamp pad 51 such as a silicon rubber sheet disposed in a cell culture dish 50. By performing incubation in this state, gelation of the liquid 110 is completed and a gel 120 is obtained (Fig. 8C). Since the gel 120 interposed between the first production device 10 and the stamp pad 51 is molded according to the shape of the concave part 17, the first structure 100 having a first shape is formed. The second structure 300 is taken into the inside of the gel 120, and thus is held by the first structure 100 having a first shape (Fig. 8D). Then, a growth medium can be supplied to the cell culture dish 50, and additional incubation can be performed.

On the other hand, in a case in which it is determined that the first structure 100 does not hold the second structure 300 (No in Step S2), the positioning step for the second structure 300 is not performed, a step (Step S6) in which the liquid 110 is supplied to the first production device 10 and a step (Step S7) in which the liquid 110 is gelled to form the first structure 100 are performed. The procedure of these steps is the same as in Step S4 and Step S5 except that the second structure 300 is not disposed in the first production device 10.

After Step S5 or Step S7, the obtained first structure 100 is removed from the first production device 10 (Step S8). For example, by taking the first production device 10 carefully with a tweezers or the like in the state of Fig. 8C, the first production device 10 and the first structure 100 can be separated from each other. However, a method of separating the first structure 100 is not limited to the above example, and any method may be used.

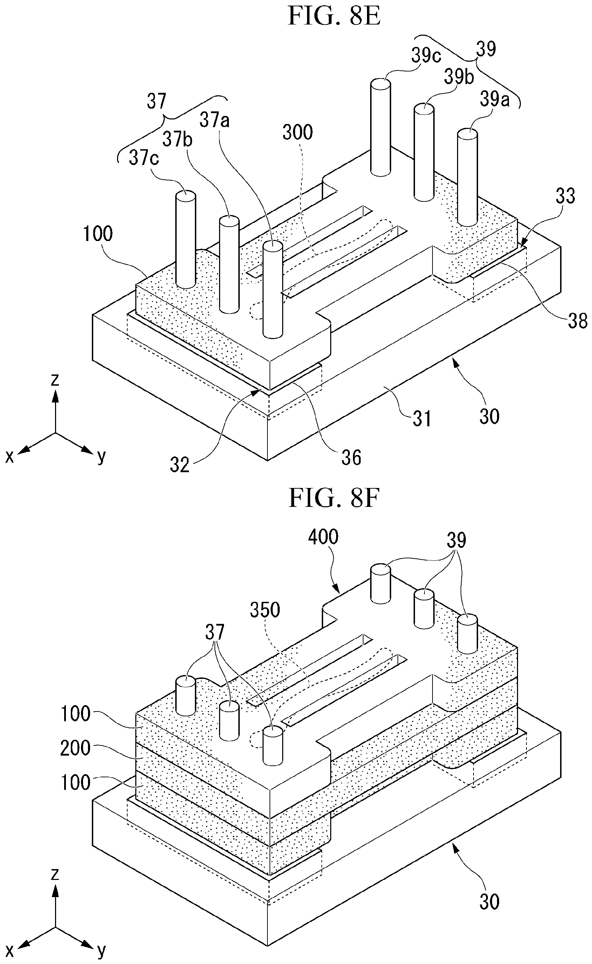

Next, the first structure 100 is attached to the first anchor member 32 and the second anchor member 33 of the third production device 30 (Step S9). For example, as shown in Fig. 8E, the anchors 37a, 37b, and 37c of the first anchor member 32 pass through the holes 104a, 104b, and 104c of the first structure 100, and the anchors 39a, 39b, and 39c of the second anchor member 33 pass through the holes 105a, 105b, and 105c of the first structure 100. Thereby, both end portions of the first structure 100 are fixed with the first anchor member 32 and the second anchor member 33. In this manner, the first structures 100 are stacked such that both end portions thereof are fixed with the anchors.

Next, it is determined whether stacking for forming a 3D tissue composite is completed (Step S10). If stacking is not completed (No in Step S10), the process returns to the step before Step S2, the next first structure is produced, and the first structure is sequentially stacked. Preferably, in the next cycle in which the first structure 100 having a first shape is produced using the first production device 10, the first structure 200 having a second shape is produced using the second production device 20, and in the next cycle in which the first structure 200 having a second shape is produced using the second production device 20, the first structure 100 having a first shape is produced using the first production device 10. That is, the first structure 100 having a first shape and the first structure 200 having a second shape are preferably alternately stacked. Thereby, in a cross-sectional view perpendicular to the x direction, the positions of the slits 106 and 206 of the first structures 100 and 200 in the y direction are different for each layer, and gaps (the slits 106 and 206) in the 3D structure obtained by stacking the first structures 100 and 200 are alternately disposed in a cross-sectional view along the x direction. That is, in the y direction which is orthogonal to the x direction and the z direction, which is the stacking direction, the slits 106 and 206 of one of two adjacent first structures 100 and 200 are provided at positions different from those of the slits 106 and 206 of the other of two adjacent first structures 100 and 200. Such a disposition is preferable particularly in a case in which a muscle tissue is formed as a first tissue because the gaps of the slits 106 and 206 are efficiently filled according to contraction of the muscle fiber bundle. However, the order of production and stacking is not limited to the above example, and as necessary, the first structures 100 and 200 may be produced and stacked in any order.

On the other hand, in a case in which it is determined in Step S10 that stacking is completed (Yes in Step S10), a stacked body including stacked first structures 100 and 200 is formed on the third production device 30. Next, in the second structure 300 having the core part 301 containing second cells or second tissues and the shell part 302 that surrounds the core part 301, the shell part 302 is dissolved (Step S11). For example, in a case in which the shell part 302 is an arginate gel, the shell part 302 can be dissociated according to the action of an enzyme such as arginase. As a method of dissociating an arginate gel, in addition to use of an enzyme, a method of removing calcium ions according to the action of a chelating reagent such as EDTA at an appropriate concentration, and a method of allowing a weak acid such as citric acid to act may be exemplified. Thereby, the shell part 302 is removed so that the cell fiber of the second cells or the tissue fiber of second tissues forming the core part 301 of the second structure 300 can come into direct contact with the first structures 100 and 200. Here, the second structure after the shell part 302 is dissociated is referred to as "a second structure 350" in order to distinguish it from the second structure 300 in which the shell part 302 is not dissociated. In this manner, a 3D composite 400 having a structure in which the first structures 100 and 200 are stacked and the second structure 350 is held by one or more first structures 100 and 200 is obtained. For example, Fig. 8F shows a 3D composite 400 having a three-layer structure in which the first structure 100 having a first shape, the first structure 200 having a second shape, and the first structure 100 having a first shape are sequentially stacked on the third production device 30.

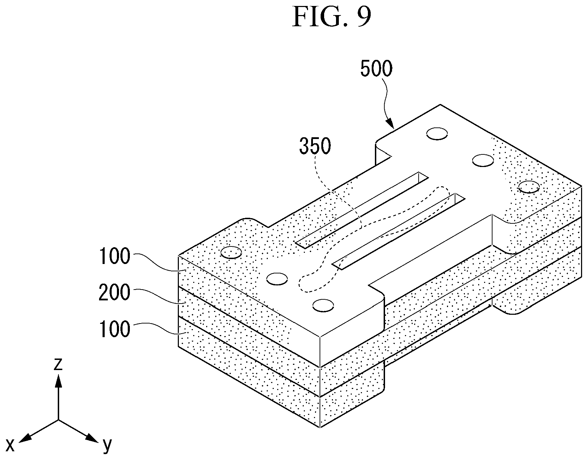

Next, the 3D composite 400 is cultured (Step S12). In the present embodiment, the first cells are myoblasts, the myoblasts are differentiated into muscle cells according to culturing using a differentiation medium and an electrical stimulus, and additionally, a muscle fiber (an example of "first tissue") is formed from muscle cells. The muscle fiber bundle can be formed along the direction of the slits 106 and 206. When this muscle fiber bundle is formed, contraction of the first tissue occurs between both end portions of the first structures 100 and 200 fixed with the anchors, and thereby gaps which was generated by dissolution of the shell part 302 of the second structure 300 are filled. Here, a general electrical stimulus culture system can be used for the electrical stimulus. By forming the first tissue in this manner, a 3D tissue composite 500 in which the first tissue and the second tissue are three-dimensionally combined is obtained (Fig. 9). Subsequently, the 3D tissue composite 500 is cultured, and thus the first tissue of the first structures 100 and 200 and the second tissue of the second structure 350 are three-dimensionally co-cultured.

In this manner, the second structure is held by the sheet-shaped first structure and the first structures are stacked to form a 3D tissue composite, and thereby it is possible to control the position of the second structure containing the second tissue in the 3D tissue composite. Thereby, in the co-culture of the first tissue and the second tissue, it is possible to control the position of the second tissue with respect to the first tissue, and it is possible to improve handling properties of the 3D tissue composite. In addition, by positioning the second structure containing second tissues so as to reproduce the in-vivo environment, it is possible to improve the equivalence of the 3D tissue composite with the living body.

Here, there may be no interval in a period after the second structure 300 is produced in Step S1 until production of the first structures 100 and 200 starts in Step S2 and the following step. However, in a case in which there is a difference in the culture period between second cells and first cells, particularly in a case in which a culture period required for second cells is longer than a culture period for first cells, after Step S1, the second cells are sufficiently cultured and Step S2 and the following step can then start. That is, the second structure 300 may be cultured in Step S1 and the following step. The culture period of the second structure 300 is not particularly limited, and may be, for example, 1 day or more, 2 days or more, 3 days or more, 1 week or more, 1 month or more, and may be, for example, 1 year or less, 6 months or less, 3 months or less, or 2 months or less. Thereby, the second structure 300 can contain second tissues formed by culturing second cells and thus co-culturing can be efficiently performed. However, before a second tissue is formed from the second cells, the 3D composite 400 is produced according to the above process and the 3D composite 400 is cultured, and thus the first cells (for example, myoblasts or muscle cells) and the second cells (for example, preadipocytes or adipocytes) may be co-cultured.

### [3D tissue composite]

Fig. 9 is a schematic view showing the 3D tissue composite 500 according to the present embodiment. The 3D tissue composite according to the present embodiment is a 3D tissue composite including a sheet-shaped first structure containing a first tissue and the second structure held by the first structure and contains a second tissue, and in which the multiple number of first structures are stacked. That is, the 3D tissue composite 500 produced by the production method has a structure in which the multiple number of sheet-shaped first structures 100 and 200 are stacked. At least one of the multiple number of first structures 100 and 200 holds the second structure 350. The first structures 100 and 200 contain a first tissue, and the second structure 350 contains a second tissue. In the present embodiment, the first tissue is a muscle tissue, the second tissue is an adipose tissue, and the 3D tissue composite 500 is a muscle-fat composite tissue. Examples of muscle tissues include a skeletal muscle tissue, a smooth muscle tissue, and a myocardial tissue.

In the present embodiment, the second structure 350 is surrounded by the first structures 100 and 200, and is at least partially embedded inside the first structures 100 and 200. In addition, it is preferable that the first tissue of the first structures 100 and 200 and the second tissue of the second structure 350 be in direct contact with each other. Thereby, the reproducibility of the in-vivo environment in which tissues are in direct contact with each other can be improved, and thus the equivalence with a living body can be improved.

In the present embodiment, the second structure 350 has an elongated fiber shape. In Fig. 9, the fibrous second structure 350 extends in the first structures 100 and 200 along the x direction. In addition, the muscle tissue constituting the first structures 100 and 200 has muscle fibers extending along the x direction. In the first structures 100 and 200, the slits 106 and 206 extending along the x direction are formed, and thus the muscle fibers extend along the x direction.

The 3D tissue composite 500 according to the present embodiment can be used for drug screening in which a drug is added, the change in the state of a constituent tissue such as a muscle tissue or an adipose tissue is observed, and thus the effect of the drug on a living body is verified. In addition, by performing a drug test while applying an electrical stimulus to the 3D tissue composite 500, it is possible to verify the change in drug responsiveness due to a contraction movement of the muscle tissue induced by the electrical stimulus. In addition, the 3D tissue composite 500 can also be used as, for example, edible artificial meat.

### [Modification examples]

In the above embodiment, the fibrous second tissue and the fibrous second structure have been exemplified, but other forms of second structures may be used. For example, the second structure may contain a bead-shaped second tissue, may be formed in a sheet shape like the first structure, or may have any other form. In a case in which a bead-shaped second tissue is used, the second structure in the form of bead may be at least partially embedded in the sheet-shaped first structure. In the positioning step (Step S3) of the second structure, the bead-shaped second structure can be disposed at an appropriate position on the holding surface of the production devices 10 and 20. In addition, in the case of forming a sheet-shaped second structure, a first structure and a second structure can be stacked on the third production device 30. For example, by interposing a sheet-shaped second structure containing second cells or second tissues between two first structures to stack them, the first structure can hold the sheet-shaped second structure. Here, the sheet-shaped second structure may be produced by the same method as the first structure of the above embodiment.

While an example in which the first cells are myoblasts, the first tissues are muscle tissues, the second cells are preadipocytes or adipocytes, and the second tissues are adipose tissues has been described in the above embodiment, these are not limited to the above example, and other cells and tissues can be used in addition to the muscle tissues and adipose tissues or in place of the muscle tissues and adipose tissues. For example, in place of the adipose tissues or in addition to the adipose tissues, other elements such as vessels may be formed in the muscle tissue by any method. For example, the second structure may contain two or more types of tissues such as adipose tissues and vascular tissues, and in addition to the first structure and the second structure, one or more additional structures such as a third structure containing other tissues such as vascular tissues may be used.

In addition, the first structure may contain two or more types of tissues. For example, a tendon-muscle composite tissue in which a tendon tissue is bonded to both end portions of a muscle tissue may be formed, by culturing tendon cells to form a tendon tissue at both end portions of the first structure and culturing myoblasts between them to form a muscle tissue. In a case in which the 3D tissue composite 500 is produced by the above production method using the first structure containing such a tendon-muscle composite tissue, if the second tissue is an adipose tissue, a tendon-muscle-fat-co-cultured tissue containing an adipose tissue in a tendon-muscle composite tissue is obtained.

While an example in which only the first walls 12 and 22 and the second walls 13 and 23 are provided on the production devices 10 and 20 as side walls has been described in the above embodiment, side walls may be additionally provided on the side of the first ends 11a and 21a and the second ends 11b and 21b of the bases 11 and 21. Then, since the concave parts 17 and 27 are surrounded from all sides, even if the liquid 110 having a low viscosity or the liquid 110 in which the progress of gelation is slow is used, it is possible to prevent the liquid 110 from flowing out from the concave parts 17 and 27.

According to at least one of the embodiments described above, by controlling the position of the second tissue with respect to the first tissue in co-culture of the first tissue and the second tissue, it is possible to improve handling properties of the 3D tissue composite and the equivalence with a living body.

### [Examples]

Hereinafter, the present invention will be described with reference to experimental examples, but the present invention is not limited to the following experimental examples.

### [Experimental Example 1]

### (Production of second structure)

According to the production procedure schematically shown in Fig. 7, a second structure containing adipocyte fibers was produced. As cells, 3T3-L1 which is a preadipocyte derived from a mouse was used, atelocollagen (DME-02H, commercially available from Koken Co., Ltd.) was added, and a cell collagen suspension (core solution) having a cell density of 1.0×10⁸ cells/mL was prepared. The cell collagen suspension was introduced at a flow rate of 100 µL/min from the inlet 41 of the microfluidic device 40, a 1.5 wt% sodium alginate solution as a shell solution was introduced at a flow rate of 300 µL/min from the inlet 42, a 3 wt% sucrose solution containing 100 mM calcium chloride which is a sheath solution for gelling alginate sodium was introduced and injected at a flow rate of 3,600 µL/min from the inlet 43, and thereby adipocyte fibers having a core part with a diameter of about 200 µm o and a shell part with an outer diameter of about 350 µm were obtained.

The obtained adipocyte fibers were cultured in a growth medium for 3 to 5 days, and proliferated so that the insides of the fibers were filled with 3T3-L1. Then, the medium was replaced with a differentiation medium, and culture was performed for 2 days. Then, the medium was replaced with a promoting medium in order to promote the growth of fat droplets of adipocytes. The cells were cultured in an incubator in which the temperature was set to 37°C, and the carbon dioxide concentration was set to 5% (the same applies hereinafter). Here, as the growth medium, a solution in which fetal bovine serum (FBS) having a volume ratio of 10% and penicillin-streptomycin having a volume ratio of 1% were mixed into DMEM (Dulbecco's modified eagle medium) Low Glucose (041-29775, commercially available from Wako Co., Ltd.) was used. As the differentiation medium, PGM TM-2 Preadipocyte Growth Medium-2 BulletKit Tm, PT-8002 (commercially available from Lonza) was used. As the promoting medium, a solution in which FBS having a volume ratio of 10%, penicillin-streptomycin having a volume ratio of 1% and 5 µg/mL of insulin were mixed into DMEM High Glucose (D5796, commercially available from Sigma-Aldrich) was used.

### [Experimental Example 2]

### (Production of first structure)

According to the production procedure schematically shown in Fig. 8A to 8D, a first structure containing myoblasts was produced. The first production device 10 and the second production device 20 were produced as a stamp made of PDMS by transferring PDMS to a mold output from an optical molding device.

As cells, C2C12 (ATCC), which is a myoblast cell line derived from a mouse, was used. A C2C12 growth medium and a thrombin solution were added at a volume ratio of 8.6:1 with respect to C2C12 in an 80% confluence state on a dish, and a mixed liquid A having a cell concentration of 5.0×10⁷ cells/mL was prepared. Next, a growth medium, Matrigel, and a fibrinogen solution were mixed at a volume ratio of 1:10:20 to prepare a mixed liquid B. After the second structure was disposed at a desired position on the first production device made of PDMS with a tweezers, 42.7 µL of the mixed liquid A and 68 µL of the mixed liquid B were mixed, and put into a concave part of the first production device, and the mixed liquid was interposed between the first production device and the silicon rubber sheet. Then, incubation was performed for 30 minutes for gelation of Matrigel. Next, the growth medium was added and culture was performed for 2 to 3 hours. Then, the first production device was taken with the tweezers, and the sheet-shaped first structure holding the second structure (refer to Fig. 8D; hereinafter referred to as a "muscle-fat composite sheet") was obtained.

Here, as the growth medium, a solution in which FBS having a volume ratio of 10% and penicillin-streptomycin having a volume ratio of 1% were mixed into DMEM High Glucose (D5796, commercially available from Sigma-Aldrich) was used. As the thrombin solution, 5 mL of a 0.1% BSA solution contained in a thrombin (T7009-250, commercially available from Sigma-Aldrich) bottle was used. The fibrin solution was prepared by dissolving fibrinogen (F8630, commercially available from Sigma-Aldrich) in a phosphate buffered saline (PBS) so that the concentration was 20 mg/mL.

### [Experimental Example 3]

### (Production of 3D structure)

According to the production procedure schematically shown in Fig. 8E and Fig. 8F, a 3D structure containing myoblasts and adipocytes was produced. The base 31 of the third production device 30 was produced by transferring PDMS to the mold output from the optical molding device, and the first anchor member 32 and the second anchor member 33 were output from the optical molding device, and parylene vapor deposition was performed on the surface. The diameter of the anchor was 600 µm, and the height was 3.5 mm.

The muscle-fat composite sheet obtained in Example 2 was fixed to the above anchor as shown in Fig. 8E. Next, in the same manner as in Example 2, one first structure having a second shape (refer to Fig. 4) different from the above first structure was produced by the second production device made of PDMS, and fixed to the anchor so as to be stacked on the first structure of Example 2. In addition, another first structure having a first shape as in Example 2 was produced by the first production device made of PDMS, and fixed to the anchor so as to be stacked on the first structure having a second shape. Thereby, three first structures in the order of first shape-second shape-first shape were fixed to the anchors.

Then, a solution in which an ACA solution for a growth medium having a volume ratio of 1% was added to a C2C12 growth medium, and additionally arginase (commercially available from Sigma-Aldrich) was added so that the concentration was 40 µg/mL was added to the 3D structure fixed to the anchor, and cultured for 90 minutes to dissolve alginate sodium constituting the shell part of the second structure. Here, the ACA solution for a growth medium was prepared by adding 3 g of ACA (6-aminocaproic acid, A2504-25G, commercially available from Sigma-Aldrich) to 20 mL of a solution in which FBS having a volume ratio of 10% and penicillin-streptomycin having a volume ratio of 1% were mixed into DMEM High Glucose. Then, replacement with a growth medium containing no arginase was performed, and culture was performed for 1 day to stabilize cells so that they adhered to the gel.

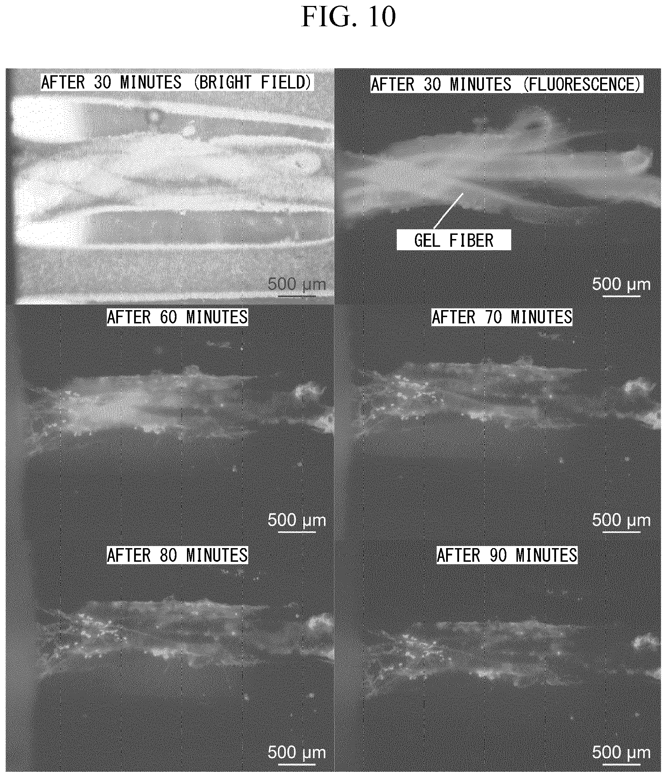

Fig. 10 shows images for observing changes over time in fat fibers after arginase was added. The upper left image is a bright-field image, and the other images are fluorescent images. The time after arginase was added is shown in the top of each image. A sheet was produced using gel fibers (second structure) in which green fluorescent beads were mixed with alginate sodium, arginase was added, and the mode in which the shell part of the second structure was dissolved was observed. A white part in the fluorescent image was a shell part composed of an arginate gel. 30 minutes after arginase was added, dissolving of the shell surface of alginate sodium was observed. In addition, 70 minutes after arginase was added, most of the shell part was dissolved. It was confirmed that the shell of alginate sodium was dissolved according to the reaction for 70 minutes or more.

### [Experimental Example 4]

### (Culture of 3D structure)

For the 3D structure obtained in Experimental Example 3, the growth medium was replaced with a medium in which an ACA solution for a differentiation medium having a volume ratio of 1% was added to the following differentiation medium for myoblasts, and differentiation of myoblasts into skeletal muscle cells was induced. Here, the differentiation medium for myoblasts was a solution in which horse serum having a volume ratio of 10% (commercially available from Sigma-Aldrich) and penicillin-streptomycin having a volume ratio of 1% were mixed into DMEM Low Glucose (041-29775, commercially available from Wako Co., Ltd.). The ACA solution for a differentiation medium was prepared by adding 4 g of ACA to 20 mL of the differentiation medium.

Next, in order to form a muscle fiber bundle from skeletal muscle cells, an electrical stimulus was applied to the 3D structure using an electrical stimulus culture system (CLD6W35F, C-Pace EM). Specifically, a sterilized electrode plate was placed in a dish containing a 3D structure and cultured in an incubator for 7 days. The electrical stimulus was applied at a voltage of 7 V, a frequency of 1 Hz, and 2 ms for 2 hours every day.

### [Experimental Example 5]

### (Electrical stimulus of 3D tissue composite)

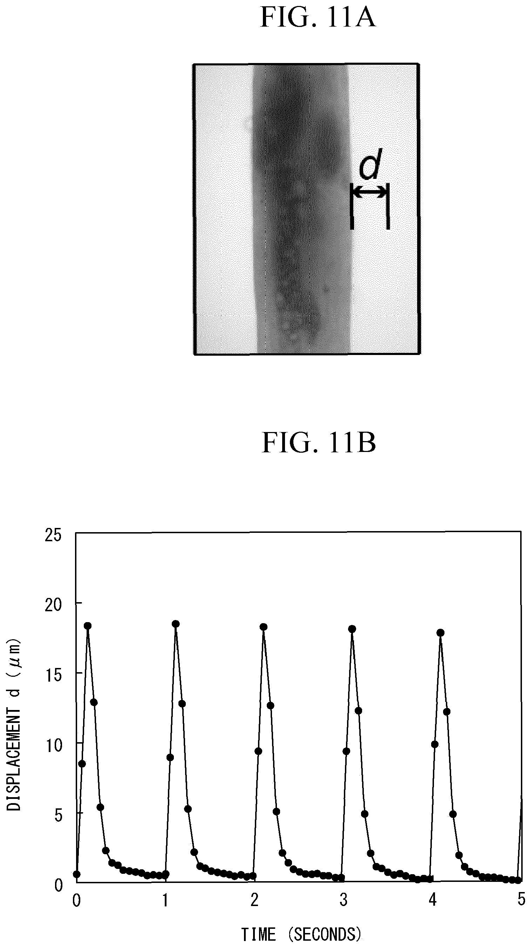

An electrical stimulus was applied to the produced muscle-fat composite sheet under the same conditions as above, and the behavior of the skeletal muscle tissue was observed. Fig. 11A is an image showing electrical stimulus responsiveness of the muscle-fat composite sheet (the vertical direction of the image is a direction in which the muscle fibers extend), and Fig. 11B is a graph showing electrical stimulus responsiveness of the muscle-fat composite sheet. While applying an electrical stimulus at a frequency of 1 Hz and 2 ms to the muscle-fat composite sheet, the muscle fiber bundle of the muscle-fat composite sheet was observed under a bright-field microscope, and the displacement d (refer to Fig. 11A) in the direction orthogonal to the extension direction of the muscle fiber bundle was measured. The change in the displacement d over time shows the graph of Fig. 11B. It was confirmed that the displacement d of the skeletal muscle tissue changed periodically according to the cycle of the electrical stimulus. In this manner, electrical stimulus responsiveness of the skeletal muscle tissue in the muscle-fat composite sheet was confirmed.

### [Experimental Example 6]

### (Position of adipose tissue)

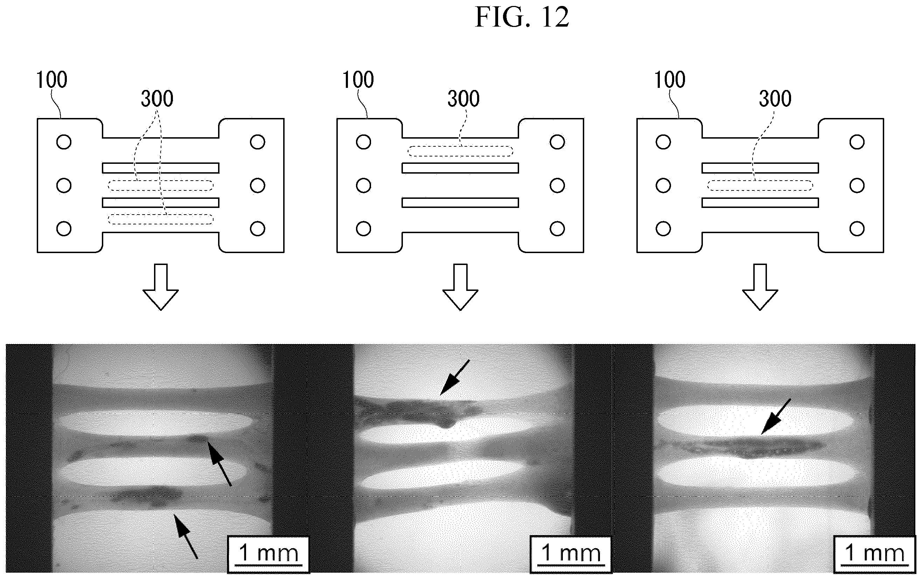

Fig. 12 shows schematic views showing positioning of the adipose tissues in the muscle-fat composite sheet (corresponding to the first shape shown in Fig. 2) and corresponding bright-field images. The upper schematic view shows the position at which the second structure is disposed during production. Black parts (at positions indicated by arrows in the drawings) in the lower bright-field image are the positions of adipocytes. From each image, it was confirmed that a part containing only skeletal muscle tissues and a part in which skeletal muscle tissues and adipose tissues were mixed were separately provided in one sheet. In addition, it was confirmed that the position of the adipocyte confirmed in the bright-field image matched the position at which the second structure (fat fiber) was disposed during production. That is, it was confirmed, by positioning the second structure during production, that it was possible to control the position of the second structure in the produced first structure.

### [Experimental Example 7]

### (Contact between skeletal muscle tissue and adipose tissue)

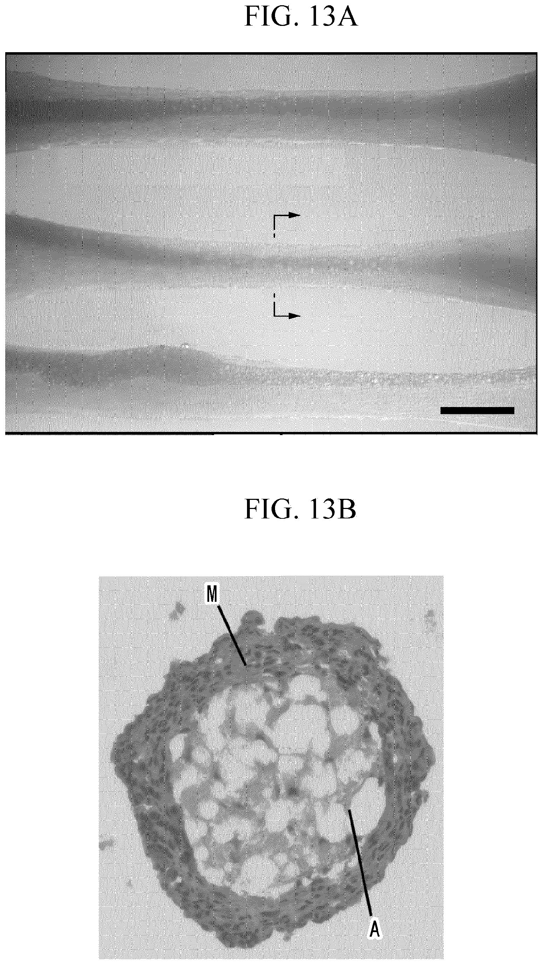

The produced muscle-fat composite sheet was frozen and cut, hematoxylin/eosin staining (HE staining) was performed, and the cut surface of the frozen section was observed. Fig. 13A is an image of the muscle-fat composite sheet showing the cut part, and Fig. 13B is an image of the HF-stained cut surface of the frozen section of the muscle-fat composite sheet. Seeing the cut surface of Fig. 13B, it was confirmed that the adipose tissue A was present at the center, the skeletal muscle tissue M was positioned to surround the adipose tissue A, and the adipose tissue A and the skeletal muscle tissue M were in contact with each other.

### [Experimental Example 8]

### (Immunostaining of section of 3D tissue composite)

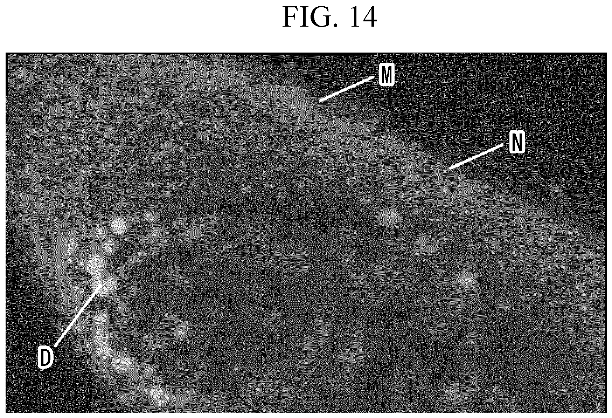

Fig. 14 shows the results obtained by immunostaining a section of the produced 3D tissue composite. As shown in Fig. 14, fat oil droplets D were observed in addition to myotubes M and nuclei N of skeletal muscle cells, and thus it was confirmed that muscle fibers and adipose tissues functioned normally in the produced 3D tissue composite.

## Claims

1. A method of producing a 3D tissue composite, comprising:
a preparation step in which a multiple number of sheet-shaped first structures containing first cells are prepared, wherein at least one of the multiple number of first structures holds a second structure containing second cells;
a stacking step in which the multiple number of first structures are stacked to form a 3D composite; and
a culturing step in which the 3D composite is cultured to form a 3D tissue composite containing first tissues formed from the first cells and second tissues formed from the second cells.

2. The method of producing a 3D tissue composite according to claim 1,
wherein the first tissues and the second tissues are co-cultured in the culturing step.

3. The method of producing a 3D tissue composite according to claim 1 or 2,
wherein the preparation step includes a positioning step in which the second structure is positioned on a substrate, and a gelation step in which a liquid containing the first cells is gelled on the substrate to form the first structure that holds the second structure.

4. The method of producing a 3D tissue composite according to any one of claims 1 to 3,
wherein the first structures are stacked such that both end portions of the first structures are fixed with anchors in the stacking step.

5. The method of producing a 3D tissue composite according to any one of claims 1 to 4,
wherein the second structure contains the second tissues formed by culturing the second cells in the preparation step.

6. The method of producing a 3D tissue composite according to any one of claims 1 to 5,
wherein the second structure has a fiber shape.

7. The method of producing a 3D tissue composite according to any one of claims 1 to 6,
wherein the second structure has a core part containing the second cells or the second tissues and a shell part that surrounds the core part, and
wherein the method further includes a dissolving step in which the shell part is dissolved.

8. The method of producing a 3D tissue composite according to any one of claims 1 to 7,
wherein the first cells are myoblasts or muscle cells, the first tissues are muscle tissues, the second cells are preadipocytes or adipocytes, and the second tissues are adipose tissues.

9. A 3D tissue composite, comprising:
a sheet-shaped first structure containing first tissues; and
a second structure held by the first structure, the second structure containing second tissues,
wherein the multiple number of first structures are stacked.

10. The 3D tissue composite according to claim 9,
wherein first tissues constituting the first structure and second tissues constituting the second structure are in contact with each other.

11. The 3D tissue composite according to claim 9 or 10,
wherein the second structure has a fiber shape.

12. The 3D tissue composite according to any one of claims 9 to 11,
wherein the second structure extends in the first structure along a first direction, and
wherein the first tissues are muscle tissues containing muscle fibers along the first direction.

13. The 3D tissue composite according to claim 12,
wherein the first structure has one or more openings extending along the first direction.

14. The 3D tissue composite according to claim 13,
wherein the opening of one of two adjacent first structures is at a position different from that of the opening of the other of the two adjacent first structures in a second direction orthogonal to the first direction and a stacking direction of the first structure.

15. The 3D tissue composite according to any one of claims 9 to 14,
wherein the first tissues are muscle tissues, and the second tissues are adipose tissues.
